# EUROPEAN PATENT SPECIFICATION

(11) **EP 2 704 675 B1**
(45) Date of publication and mention of the grant of the patent: **17.08.2016**
(21) Application number: 12782233.6
(22) Date of filing: 01.05.2012
(51) Int. Cl.: A61F 13/15, A61F 13/84, A61F 13/47

(54) **ARTICLE INCLUDING VISUAL SIGNAL FOR COMMUNICATION OF DEPTH PERCEPTION**
ARTIKEL MIT VISUELLEN SIGNALEN ZUR ÜBERMITTLUNG EINER TIEFENWAHRNEHMUNG
ARTICLE COMPRENANT UN SIGNAL VISUEL POUR LA COMMUNICATION DE LA PERCEPTION DE PROFONDEUR

(30) Priority: 06.05.2011 CN 201110125981
(43) Date of publication of application: 12.03.2014
(73) Proprietor: Johnson & Johnson Consumer Inc., Skillman, NJ 08558 (US)
(72) Inventor: CASTANARES, Karen Grace, B., Shanghai 201103 (CN); SHAOJUN, Mei, Yangzhou City Jiangsu (CN); MARCELO, Ana Maria, Elena, Singapore 589627 (SG)
(74) Representative: Carpmaels & Ransford LLP
(86) International application number: PCT/US2012/035938
(87) International publication number: WO 2012/154444

(56) References cited:
- US-A1- 2003 114 811
- US-A1- 2006 111 684
- US-A1- 2009 157 021
- US-A1- 2009 157 021
- US-A1- 2009 281 513
- US-A1- 2010 286 644
- US-A1- 2011 028 929
- US-B2- 7 241 280

## Description

### FIELD OF INVENTION

The present invention generally relates to an absorbent article including a plurality of non-colored portions and a plurality of colored portions having a single color, the plurality of non-colored portions and colored portions cooperating to create a perception of depth by a user viewing a top surface of the absorbent article.

### BACKGROUND OF THE INVENTION

Due to improvements in materials that constitute disposable sanitary absorbent articles (such as sanitary napkins, pantiliners, and the like), and improvements in the construction of such articles, commercially available disposable sanitary absorbent articles can provide excellent fluid handling properties. Further, it is known in the prior art to provide disposable absorbent articles with other functional properties. For example, it is known to provide the cover layer of such articles with a skin soothing agent to thereby provide a skin-soothing benefit to such articles. However, much of the technology that provides enhanced fluid handling properties, or other functional properties to the article, is hidden from the consumer. Consequently, these enhanced functional properties are not readily communicated to the user, visually or otherwise.

Communication of enhanced functioning characteristics by creating the perception of depth within an absorbent article is one way to solve the above described problem. Attempts have been made in the prior art to communicate depth perception by way of the use of multiple shades (i.e. tones) or multiple colors. One such attempt is described in U.S. Patent No. 7,163,526. A problem with creating depth perception by way of multiple shades and/or multiple colors is that it may increase manufacturing costs.

In view of the above, a unique absorbent article structure is disclosed herein wherein the perception of depth is provided by a plurality of non-colored portions and a plurality of colored portions having a single color, the plurality of non-colored portions and colored portions cooperating to create a perception of depth by a user viewing the top surface of the absorbent article.

### SUMMARY OF THE INVENTION

In view of the foregoing, the present invention provides an absorbent article including a longitudinally extending axis, a transversely extending axis, a top surface, a cover layer, a barrier layer, an absorbent core arranged between the cover layer and the barrier layer, a plurality of colored portions, each of the colored portions having a color that is the same as all of the other colored portions, and a plurality of non-colored portions, wherein the plurality of colored portions and the plurality of non-colored portions are arranged to create a perception of depth within the absorbent article by a user looking upon the top surface of the absorbent article, and each colored portion and non-colored portion includes at least a portion thereof that has a width of at least 6.4mm (0.25 inch).

### BRIEF DESCRIPTION OF THE DRAWINGS

Examples of embodiments of the present invention will now be described with reference to the drawings, in which:
Fig. 1 is a top perspective view of an absorbent article according to the present invention;
   and
Fig. 2 is an exploded view of the absorbent article shown in Fig. 1.

### DETAILED DESCRITION OF THE INVENTION

The present invention generally relates to disposable absorbent articles such as sanitary napkins, pantiliners, absorbent products for incontinence, baby diapers, and other disposable absorbent articles adapted to absorb body exudates. The term "disposable" as used herein refers to absorbent articles that are not intended to be laundered or otherwise reused as an absorbent article. Although the invention will be described herein with reference to a sanitary napkin, the invention may be utilized with other disposable absorbent articles.

The color scale values, utilized herein, are determined using the widely accepted CIE LAB scale. Measurements are made with a Hunter Color reflectance meter. A complete technical description of the system can be found in an article by R. S. Hunter, 'Photoelectric Color Difference Meter', Journal of the Optical Society of America, Vol. 48, pp.985-95, 1958. Devices specially designed for the measurement of color using the Hunter scales are described in U.S. Pat. No. 3,003,388 to Hunter et al., issued Oct. 10, 1961. In general, Hunter Color "L" scale values are units of light reflectance measurement, and the higher the value is, the lighter the color is since a lighter colored material reflects more light. In particular, in the Hunter Color system the "L" scale contains 100 equal units of division. Absolute black is at the bottom of the scale (L=0) and absolute white is at the top of the scale (L=100). Thus in measuring Hunter Color values of the materials used in the absorbent articles according to the present invention, the lower the "L" scale value, the darker the material and the higher the "L" scale value, the lighter the material.

Colors can be measured according to an internationally recognized 3D solid diagram of colors where all colors that are perceived by the human eye are converted into a numerical code. The CIE LAB system is similar to Hunter L, a, b and is based on three dimensions, specifically L*, a*, and b*.

When a color is defined according to this system L* represents lightness (0=black, 100=white), a* and b* independently each represent a two color axis, a* representing the axis red/green (+a=red,-a=green), while b* represents the axis yellow/blue (+b=yellow,-b=blue). A color may be identified by a unique ΔE* value (i.e., different in color from some standard or reference), which is mathematically expressed by the equation: Δ E*=[(L*_{X}.-L*_{Y})²+(a*_{X}.-a*_{Y})²+( b*_{X}-b*_{Y})²]^{1/2} 'X' represents the standard or reference sample which may either be a 'white' sample or a 'colored' sample, e.g., one color may be compared to another color. It is to be understood that the tristimulus color values and ΔE* considered herein are those measured on the materials of interest (e.g., the colored and non-colored portions viewable from the top surface of the absorbent article described herein).

The Hunter color meter quantitatively determines the amount (percent) of incident light reflected from a sample onto a detector. The instrument is also capable of analyzing the spectral content of the reflected light (e.g., how much red/green or yellow/blue is in the samples). The Hunter color meter is configured to yield three values (L*, a*, b*). The L* value is simply the percent of the incident (source) light that is reflected off a target sample to the detector. A shiny white sample will yield an L* value near 100 while a dull black sample will yield an L* value of about 0. The a* and b* value contains spectral information for the sample.

The color measurement method described in greater detail below ("Color Measurement from the Top Surface of the Article") is conducted using a Labscan XE 45/0 geometry instrument to measure the colored portions and non-colored portions of absorbent articles described herein. A 0.1875 inch diameter port on the instrument is used in the measurement method, said port having a 0.125 inch area view. The instrument was calibrated using standard white and black tiles supplied by the instrument manufacturer. During measurement the port should be arranged entirely within either the colored portion or non-colored portion being measured, i.e. during measurement the port should not overlap both a colored and non-colored portion. In order to insure that the port can be positioned entirely within the colored portion or non-colored portion each colored portion and non-colored portion must include at least a portion thereof that has a width of at least 6.4mm (0.25 inch).

The term "color" as used herein includes any color in the visual spectrum, including white, black, red, blue, violet, orange, yellow, green, indigo, as well as any mixture or combination thereof, provided such color has at least one of a L* value, a*, or b* value outside the values specified in the "non-colored" definition provided below.

The term "non-colored" or "non-color" as used herein refers to specific shades of white wherein such shades have a L* value of at least 90, an a* value equal to 0 +/- 2 and b* value equal to 0 +/- 2.

Two colors are considered to be the "same color", as such term is used herein, wherein people would not readily see a color difference between the two colors. Typically, people do not see a color difference if the ΔE* between the two colors is less than 2.

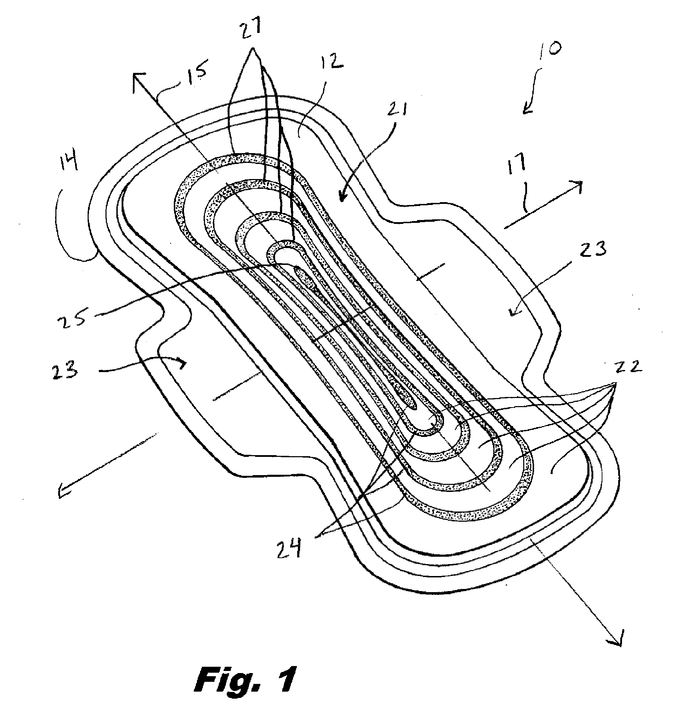

Fig. 1 provides a perspective view of a sanitary napkin 10 in accordance with one embodiment of the present invention. The sanitary napkin 10 includes a top surface 12 and a bottom surface 14. The sanitary napkin 10 further includes a longitudinally extending centerline 15 and a transversely extending centerline 17.

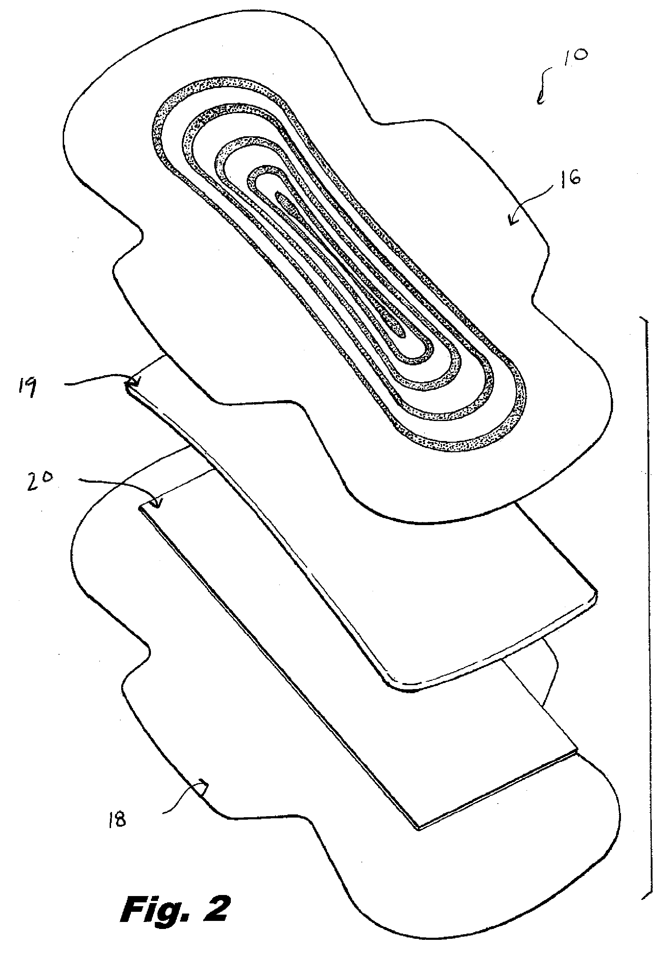

As shown in Fig. 2, the sanitary napkin 10 includes a liquid permeable cover layer 16, a liquid impermeable barrier layer 18, and an absorbent core 20 arranged between the cover layer 16 and the barrier layer 18. The sanitary napkin 10 may optionally include a transfer layer 19 arranged between the cover layer 16 and the core 20. As shown in Fig. 1, the sanitary napkin 10 includes a main body 21, which includes the primary absorbent portions of the napkin, and a pair of wings 23 that extend outwardly from the main body 21.

As shown in Fig. 1, the sanitary napkin 10 includes a plurality of non-colored portions 22 and a plurality of colored portions 24, each of the colored portions 24 having a color that is the same color as all of the other colored portions 24. Preferably each of the non-colored portions 22 has a color that is the same color as all of the other non-colored portions 22. The non-colored portions 22 and the colored portions 24 are viewable from the top surface 12 of the sanitary napkin 10. The non-colored portions 22 and colored portions 24 cooperate to create a perception of depth by a user looking upon the top surface 12 of the sanitary napkin 10.

In one embodiment of the invention, as shown in Fig. 1, the plurality of colored portions 24 includes a central colored portion 25 that is located substantially at the center of the napkin 10 and extends generally in a longitudinal direction of the napkin. The central colored portion 25 is arranged such it that is symmetrical to the longitudinally extending centerline 15 and transversely extending centerline 17. The central colored portion 25 generally has a slender peanut type shape and the central colored portion 25 is surrounded by a plurality of peanut shape rings 27 that surround the central colored portion 25. Moving outward from the intersection of the longitudinally extending centerline 15 and transversely extending centerline 17, each ring 27 is larger than the previous ring 27. In this way, the rings 27 radiate outward from the central colored portion 25. Each one of the rings 27 is spaced from a previous ring 27 by one of the non-colored portions 22. In this way each one of the rings 27 is arranged in spaced relationship to an adjacent ring 27 and completely surrounds a previous ring 27. In addition, the first ring 27, i.e. the ring closest to the central colored portion 25, completely surrounds the central colored portion 25.

In the embodiment of the invention shown in Fig. 1, the plurality of non-colored portions 22 and the plurality of colored portions 24 are concentrically arranged such that they radiate outwardly from the intersection of the longitudinally extending centerline 15 and transversely extending centerline 17. In the embodiment of the invention shown in Fig. 1, the plurality of non-colored portions 22 and colored portions 24 are concentrically arranged such that a center of the concentric arrangement is located at the intersection of the longitudinally extending centerline 15 and transversely extending centerline 17. Of course, the arrangement of the plurality of non-colored portions 22 and colored portions 24 can be varied somewhat such that the center of the concentric arrangement is located at a different location than the intersection of the longitudinally extending centerline 15 and transversely extending centerline 17.

The sanitary napkin 10 preferably includes between about 3-7 colored portions 24 and each colored portion preferably has a width in the range of about 0.25 inch to about 0.60 inch. Preferably the non-colored portions 22 extend over a surface area of between about 81.6% and about 91.2% of a total surface area of the top surface 12 of the main body 21 (i.e. not including the top surface 12 of the wings 23). Preferably the colored portions 24 extend over a surface area of between about 8.8% and about 18.4% of a total surface area of the top surface of the main body 21.

The color of each of the non-colored portions 22 and the color of each of the colored portions 24 are measured using the method described herein below. As noted above, each of the colored portions 24 has a color that is the same color as all of the other colored portions 24. Preferably each of the non-colored portions 22 has a color that is the same color as all of the other non-colored portions 22. Preferably, the only colored portions of the napkin, are the colored portions 24 having the same color. Thus, preferably, the napkin 10 according to the present invention is provided with only a single color, i.e. the color of the colored portions 24. The difference in color (ΔE*) between each of the non-colored portions 22 and each of the colored portions 24 should be at least 3.5.

The color of each of the non-colored portions 22 and the color of each of the colored portions 24 are measured from the top surface 12 of the napkin 10 by a reflectance spectrophotmeter according to the method set forth below, to thereby determine the L*, a* and b* values for all of the non-colored portions 22 and colored portions 24 located within the main body 21 of the napkin 10 (i.e. excluding the areas defined by the wings 23).

Each of the non-colored portions 22 should have an L* value of at least 90, an a* value equal to 0 +/- 2 and a b* value equal to 0 +/- 2, and any color difference between individual non-colored portions 22 should have a ΔE* value of less than 2.

Each of the colored portions 24 should have a ΔE* value of less than 2 compared to each of the other colored portions 24. The difference in color (ΔE*) between each of the non-colored portions 22 and each of the colored portions 24 should be at least 3.5.

The colored portions 24 may be delivered to the sanitary napkin 10 by any one of or combination of the following: (1) color provided on the top surface 12 (i.e. body facing surface) of the cover layer 16; (2) color provided on the transfer layer 19 or core 20; (3) color provided on an insert layer arranged between the cover layer 16 and the transfer layer 19 or core 20 (if the transfer layer is omitted). The insert layer may comprise a printed tissue layer or the like. Of course, if the colored portions 24 are delivered by way of any layer located below the cover, the cover should be selected such that the colored portions 24 are visible from the top surface 12 of the napkin 10.

The colored portions 24 may be provided by any known means in the art including ink-jet printing, flexographic printing or any other suitable printing technique known to those of skill in the art.

### Cover Layer

The cover layer 16 may be a relatively low density, bulky, high-loft non-woven web material. The cover layer 16 may be composed of only one type of fiber, such as polyester or polypropylene or it may include a mixture of more than one fiber. The cover 16 may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the cover layer 16 has a basis weight in the range of about 10 gsm to about 75 gsm.

Bi-component fibers may be made up of a polyester layer and a polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layers of the article and/or to the barrier layer.

The cover layer 16 preferably has a relatively high degree of wettability, although the individual fibers comprising the cover may not be particularly hydrophilic. The cover material should also contain a great number of relatively large pores. This is because the cover layer 16 is intended to take-up body fluid rapidly and transport it away from the body and the point of deposition. Therefore, the cover layer contributes little to the time taken for the napkin to absorb a given quantity of liquid (penetration time).

Advantageously, the fibers which make up the cover layer 16 should not lose their physical properties when they are wetted, in other words they should not collapse or lose their resiliency when subjected to water or body fluid. The cover layer 16 may be treated to allow fluid to pass through it readily. The cover layer 16 also functions to transfer the fluid quickly to the underlying layers of the napkin. Thus, the cover layer 16 is advantageously wettable, hydrophilic and porous. When composed of synthetic hydrophobic fibers such as polyester or bi-component fibers, the cover layer 16 may be treated with a surfactant to impart the desired degree of wettability.

Alternatively, the cover layer 16 can be made of a polymer film having large pores. Because of such high porosity, the film accomplishes the function of quickly transferring body fluid to the underlying absorbent layers.

The cover layer 16 may be attached to the underlying absorbent core 20, transfer layer 19, and/or the barrier layer 18, by adhesion and/or other suitable means know to those of skill in the art.

### Absorbent Core

In one embodiment, the absorbent core 20 is a blend or mixture of cellulosic fibers and superabsorbent disposed therein. Cellulosic fibers that can be used in the absorbent core 20 are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Pat. No. 5,916,670 to improved flexibility of the product. Flexibility of the material may also be improved by mechanically working the material or tenderizing the material.

The absorbent core 20 can contain any superabsorbent polymer (SAP) which are well known in the art. For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc.. In a specific example, the absorbent core is a material containing from 95% to about 40% percent cellulosic fiber by weight, and about 5% to about 60% SAP by weight.

### Transfer Layer

Adjacent to cover layer 16 layer on its inner side is the optional transfer layer 19. The transfer layer 19 provides the means of receiving body fluid from the cover layer 16 and holding it until the absorbent core 20 has an opportunity to absorb the fluid, and therefore serves as a fluid transfer or acquisition layer. In addition the transfer layer 19 functions to wick the fluid in the longitudinal and transverse directions of the napkin so that the full absorbent capacity of the napkin is utilized.

The transfer layer 19 preferably has a larger proportion of smaller pores than the cover layer 16. These attributes allow the transfer layer 19 to contain body fluid and hold it away from the outer side of the cover layer 16, thereby preventing the fluid from re-wetting the cover layer 16 and its surface.

The transfer layer 19 may be composed of fibrous materials, such as wood pulp, polyester, rayon, flexible foam, or the like, or combinations thereof. The transfer layer 19 may also comprise thermoplastic fibers for the purpose of stabilizing the layer and maintaining its structural integrity. The transfer layer 19 may be treated with surfactant on one or both sides in order to increase its wettability, although generally the transfer layer 19 is relatively hydrophilic and may not require treatment. The transfer layer 19 is preferably bonded on both sides to the adjacent layers, i.e. the absorbent core 20 and the barrier layer 18.

### Barrier Layer

Underlying the absorbent core 20 is a barrier layer 18 comprising liquid-impervious film material so as to prevent liquid from egressing the sanitary napkin and staining the wearer's undergarment. The barrier layer 18 is preferably made of polymeric film, although it may be made of liquid impervious, air-permeable material such as repellent-treated non-woven or micropore films or foams.

The barrier layer 18 may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 16 and the barrier layer 18 are preferably joined along their marginal portions so as to form an enclosure or flange seal that maintains the transfer layer 19 and absorbent core 20 captive. The joint may be made by means of adhesives, heat-bonding, ultrasonic bonding, radio frequency sealing, mechanical crimping, and the like and combinations thereof.

Positioning adhesive may be applied to the bottom surface 14 of the barrier layer 18 for securing the napkin 10 to a garment during use. The positioning adhesive may be covered with removable release paper so that the positioning adhesive is covered by the removable release paper prior to use.

### Color Measurement from the Top Surface of the Article

To measure the L*, a*, and b* values of the colored portions 24 and non-colored portions 22 within the main body 21 of the sanitary napkin 10, a standard procedure is used. The color of each of the non-colored portions and the colored portions is measured using a standard procedure in accordance with the method ASTM E 1164-94, "Standard Practice for Obtaining Spectrophotometric Data for Object-Color Evaluation". This standard method is followed but specific instrument settings and sampling procedure are given herein for clarity. Sample color is reported in terms of the CIE 1976 color coordinate standard as specified in ASTM E 1164-94 and ASTM D2264-93, section 6.2. This consists of three values; L* which measures sample "lightness", a* which measures redness or greenness, and b* which measures yellowness or blueness.

**Apparatus**

| | |
|---|---|
| Reflectance | 45°/0° Hunter Labscan XE, or equivalent |
| Spectrophotometer | HunterLab Headquarters, 11491 Sunset Hills Road, Reston, VA 20190-5280 |
| Standard Plate | Standard Hunter White Tile Source: Hunter Color. |

### Equipment Preparation

1. Assure that the Spectrophotometer is configured as follows:

| | |
|---|---|
| Illumination | Type C |
| Standard Observer | 2° |
| Geometry | 45°/0° Measurement angle |
| Port Diameter | 0.1875 inch |
| Viewing Area | 0.125 inch |
| UV Filter | Nominal |

2. Calibrate the Spectrophotometer using standard black and white tiles supplied with the instrument according to the manufacturer's instructions before beginning any testing.

### Sample Preparation

1. Unwrap, unfold and lay the product or pad samples without touching or altering the color of the top surface (i.e. body facing surface) 12 of the article.
2. Areas on the top surface 12 of the article should be selected for measurement and must include the following:
   (a) Each of the non-colored portions located within the boundaries of the main body 21;
   (b) Each of the colored portions located within the boundaries of the main body 21;

### Test Procedure

1. Operate the Hunter Colorimeter according to the instrument manufacturer's instructions.
2. Pads should be measured laying flat over the 0.50 inch aperture on the instrument. A white tile should be placed behind the pad.
3. The pad should be placed with its long direction perpendicular to the instrument.
4. Measure the non-colored portions and colored portions of the article.
5. Measure the same non-colored portions and colored portions for at least 3 replicate samples.

### Calculations

1. Ensure that the readings are in CIE L*, a*, b*.
2. Record the L*, a*, b* values to the nearest 0.1 units.
3. Calculate an average L*, a* and b* for each of the non-colored portions and colored portions measured.
4. Using the above averages, calculate the ΔE* between a selected one of the non-colored portions and all of the other non-colored portions. The specific non-colored portion selected for purposes of comparison is selected by the person conducting the test.
5. Calculate the ΔE* between a selected one of the colored portions and all of the other colored portions. The specific colored portion selected for purposes of comparison is selected by the person conducting the test.
6. Determine an average L*, a* and b* for all of the colored portions based upon the values set forth in Table 1 below and an average L*, a* and b* for all of the non-colored portions set forth in Table 1 below and based upon these values determine and average ΔE* between the non-colored and colored portions.

In accordance with the present invention the ΔE* between the selected non-colored portion and all of the other non-colored portions is less than 2. In accordance with the present invention the ΔE* between the selected colored portion and all of the other colored portions is less than 2. In accordance with the invention the average ΔE* between the non-colored and colored portions is at least 3.5.

The color readings for a sanitary napkin 10 as described herein and shown in Figs. 1 and 2 are provided below. Table 1 sets forth the average values of each of the colored and non-colored regions from three napkins. Table 2 sets for the Delta E* between the selected non-colored portion (colored portion 1) and each of the other non-colored portions. Table 3 sets forth the Delta E* between the selected colored portion (portion 1) and each of the other colored portions. Table 4 sets forth the Delta E* between the selected non-colored portion and the selected colored portion.

**TABLE 1**

| | L* | a* | b* |
|---|---|---|---|
| Non-colored _{1 Average} | 92.1 | -0.7 | 0.2 |
| Non-colored _{2 Average} | 93.3 | -0.7 | 0.3 |
| Non-colored _{3 Average} | 92.7 | -0.6 | 0.6 |
| Non-colored _{4 Average} | 92.8 | -0.6 | 0.4 |
| Non-colored _{5 Average} | 93.1 | -0.7 | 0.5 |
| Colored _{1 Average} | 85.4 | -3.2 | -5.1 |
| Colored _{2 Average} | 86.1 | -2.9 | -4.6 |
| Colored _{3 Average} | 86.5 | -3.0 | -5.8 |
| Colored _{4Average} | 86.1 | -3.3 | -5.5 |
| Colored _{5 Average} | 86.8 | -2.9 | -5.6 |

**TABLE 2**

| | Delta E* |
|---|---|
| Non-colored ₁₋₂ | 1.2 |
| Non-colored ₁₋₃ | 0.7 |
| Non-colored ₁₋₄ | 0.7 |
| Non-colored ₁₋₅ | 1.0 |

**TABLE 3**

| | Delta E* |
|---|---|
| Colored ₁₋₂ | 0.9 |
| Colored ₁₋₃ | 1.3 |
| Colored ₁₋₄ | 0.8 |
| Colored ₁₋₅ | 1.5 |

**TABLE 4**

| | Delta E* |
|---|---|
| Colored ₐᵥₑ Non-colored ₐᵥₑ | 9.1 |

## Claims

1. An absorbent article comprising:
a longitudinally extending axis;
a transversely extending axis;
a top surface;
a cover layer;
a barrier layer;
an absorbent core arranged between the cover layer and the barrier layer;
a plurality of colored portions, each of the colored portions having a color that is the same as all of the other colored portions; and
a plurality of non-colored portions;
wherein the plurality of colored portions and the plurality of non-colored portions are arranged to create a perception of depth within the absorbent article by a user looking upon the top surface of the absorbent article and wherein each colored portion and non-colored portion includes at least a portion thereof that has a width of at least 6.4mm (0.25 inch).

2. The absorbent article according to claim 1, wherein each of the non-colored portions has a color that is the same as all of the other non-colored portions

3. The absorbent article according to claim 2, wherein each of the colored portions has ΔE* relative to all of the other colored portions of less than 2.

4. The absorbent article according to claim 3, wherein each of the non-colored portions has ΔE* relative to all of the other non-colored portions of less than 2.

5. The absorbent article according to claim 4, wherein a ΔE* between each of the colored portions and each of the non-colored portions is at least 3.5.

6. The absorbent article according to claim 1, wherein the plurality of colored portions includes a central colored portion and plurality of colored rings that surround the central colored portion.

7. The absorbent article according to claim 6, wherein each of the colored rings is spaced from a previous ring by one of the non-colored portions.

8. The absorbent article according to claim 7, wherein the colored rings are arranged such that they radiate outward from the central colored portion.

9. The absorbent article according to claim 8, wherein a first ring of the plurality of colored rings completely surrounds the central colored portion.

10. The absorbent article according to claim 9, wherein each colored ring is larger than a previous ring.

11. The absorbent article according to claim 10, wherein each colored ring entirely surrounds a previous colored ring.

12. The absorbent article according to claim 1, wherein the colored portions extend over a surface area of between about 8.8% and about 18.4% of a total surface area of the napkin.

13. The absorbent article according to claim 13, wherein the non-colored portions extend over a surface area of between about 81.6% and about 91.2% of a total surface area of the napkin.

14. The absorbent article according to claim 1, wherein the plurality of non-colored portions and plurality of colored portions are concentrically arranged such that they radiate outward from substantially a center of the absorbent article.

## Patentansprüche

1. Absorbierender Gegenstand, umfassend:
eine longitudinal verlaufende Achse;
eine transversal verlaufende Achse;
eine obere Oberfläche;
eine Deckschicht;
eine Barriereschicht;
einen absorbierenden Kern, der zwischen der Deckschicht und der Barriereschicht angeordnet ist;
eine Vielzahl von gefärbten Teilen, wobei jeder der gefärbten Teile eine Farbe aufweist, die gleich wie jene aller anderen gefärbten Teile ist; und
eine Vielzahl von nichtgefärbten Teilen;
wobei die Vielzahl von gefärbten Teilen und die Vielzahl von nichtgefärbten Teilen angeordnet sind, um einen Eindruck von Tiefe innerhalb des absorbierenden Gegenstands durch einen Anwender, der auf die obere Oberfläche des absorbierenden Gegenstands blickt, zu erzeugen, und wobei jeder gefärbte Teil und nichtgefärbte Teil wenigstens einen Teil davon umfasst, der eine Breite von wenigstens 6,4 mm (0,25 Inch) aufweist.

2. Absorbierender Gegenstand gemäß Anspruch 1, wobei jeder der nichtgefärbten Teile eine Farbe aufweist, die die gleiche wie jener aller anderen nichtgefärbten Teile ist.

3. Absorbierender Gegenstand gemäß Anspruch 2, wobei jeder der gefärbten Teile einen ΔE*-Wert relativ zu allen anderen gefärbten Teilen von weniger als 2 aufweist.

4. Absorbierender Gegenstand gemäß Anspruch 3, wobei jeder der nichtgefärbten Teile einen ΔE*-Wert relativ zu allen anderen nichtgefärbten Teilen von weniger als 2 aufweist.

5. Absorbierender Gegenstand gemäß Anspruch 4, wobei der ΔE*-Wert zwischen jedem der gefärbten Teile und jedem der nichtgefärbten Teile wenigstens 3,5 beträgt.

6. Absorbierender Gegenstand gemäß Anspruch 1, wobei die Vielzahl von gefärbten Teilen einen zentralen gefärbten Teil und eine Vielzahl von gefärbten Ringen umfasst, die den zentralen gefärbten Teil umgeben.

7. Absorbierender Gegenstand gemäß Anspruch 6, wobei jeder der gefärbten Ringe von dem vorhergehenden Ring durch einen der nichtgefärbten Teile getrennt ist.

8. Absorbierender Gegenstand gemäß Anspruch 7, wobei die gefärbten Ringe so angeordnet sind, dass sie von dem zentralen gefärbten Teil nach außen strahlen.

9. Absorbierender Gegenstand gemäß Anspruch 8, wobei ein erster Ring der Vielzahl von gefärbten Ringen den zentralen gefärbten Teil vollständig umgibt.

10. Absorbierender Gegenstand gemäß Anspruch 9, wobei jeder gefärbte Ring größer als der vorhergehende Ring ist.

11. Absorbierender Gegenstand gemäß Anspruch 10, wobei jeder gefärbte Ring einen vorhergehenden gefärbten Ring vollständig umgibt.

12. Absorbierender Gegenstand gemäß Anspruch 1, wobei sich die gefärbten Teile über eine Oberfläche von zwischen etwa 8,8 % und etwa 18,4 % der Gesamtoberfläche der Binde erstrecken.

13. Absorbierender Gegenstand gemäß Anspruch 13, wobei sich die nichtgefärbten Teile über eine Oberfläche von zwischen etwa 81,6 % und etwa 91,2 % der Gesamtoberfläche der Binde erstrecken.

14. Absorbierender Gegenstand gemäß Anspruch 1, wobei die Vielzahl von nichtgefärbten Teilen und die Vielzahl von gefärbten Teilen konzentrisch angeordnet sind, so dass sie im Wesentlichen von dem Zentrum des absorbierenden Gegenstands nach außen strahlen.

## Revendications

1. Article absorbant, comprenant:
un axe s'étendant de façon longitudinale;
un axe s'étendant transversalement;
une surface supérieure;
une couche de couverture;
une couche de barrière;
un noyau absorbant agencé entre la couche de couverture et la couche de barrière;
une pluralité de parties colorées, chacune des parties colorées présentant une couleur qui est la même que celle de toutes les autres parties colorées; et
une pluralité de parties non colorées;
dans lequel la pluralité de parties colorées et la pluralité de parties non colorées sont disposées de manière à créer une perception de profondeur à l'intérieur de l'article absorbant pour un utilisateur qui regarde sur la surface supérieure de l'article absorbant, et dans lequel chaque partie colorée et chaque partie non colorée présente au moins une partie de celle-ci dont la largeur est d'au moins 6,4 mm (0,25 pouce).

2. Article absorbant selon la revendication 1, dans lequel chacune des parties non colorées présente une couleur qui est la même que celle de toutes les autres parties non colorées.

3. Article absorbant selon la revendication 2, dans lequel chacune des parties colorées présente un ΔE* par rapport à toutes les autres parties colorées qui est inférieur à 2.

4. Article absorbant selon la revendication 3, dans lequel chacune des parties non colorées présente un ΔE* par rapport à toutes les autres parties non colorées qui est inférieur à 2.

5. Article absorbant selon la revendication 4, dans lequel un ΔE* entre chacune des parties colorées et chacune des parties non colorées est d'au moins 3,5.

6. Article absorbant selon la revendication 1, dans lequel la pluralité de parties colorées comprend une partie colorée centrale et une pluralité d'anneaux colorés qui entourent la partie colorée centrale.

7. Article absorbant selon la revendication 6, dans lequel chacun des anneaux colorés est espacé d'un anneau précédent par une des parties non colorées.

8. Article absorbant selon la revendication 7, dans lequel les anneaux colorés sont disposés de telle sorte qu'ils rayonnent vers l'extérieur à partir de la partie colorée centrale.

9. Article absorbant selon la revendication 8, dans lequel un premier anneau de la pluralité d'anneaux colorés entoure complètement la partie colorée centrale.

10. Article absorbant selon la revendication 9, dans lequel chaque anneau coloré est plus grand qu'un anneau précédent.

11. Article absorbant selon la revendication 10, dans lequel chaque anneau coloré entoure entièrement un anneau coloré précédent.

12. Article absorbant selon la revendication 1, dans lequel les parties colorées s'étendent sur une aire de surface qui est comprise entre environ 8,8 % et environ 18,4 % d'une aire de surface totale de la serviette.

13. Article absorbant selon la revendication 13, dans lequel les parties non colorées s'étendent sur une aire de surface qui est comprise entre environ 81,6 % et environ 91,2 % d'une aire de surface totale de la serviette.

14. Article absorbant selon la revendication 1, dans lequel la pluralité de parties non colorées et la pluralité de parties colorées sont disposées de façon concentrique de telle sorte qu'elles rayonnent vers l'extérieur à partir de sensiblement un centre de l'article absorbant.
